# EUROPEAN PATENT APPLICATION

(11) **EP 4 144 865 A1**
(43) Date of publication of application: **08.03.2023**
(21) Application number: 21886939.4
(22) Date of filing: 01.11.2021
(51) Int. Cl.: C12Q 1/6883, C12Q 1/6888, C12N 9/22, C12N 15/113

(54) **MICROPARTICLE PROBE FOR ISOLATING AND DETECTING NUCLEIC ACID FOR MULTIPLE DIAGNOSTICS**

(30) Priority: 02.11.2020 KR 20200144300
(71) Applicant: Ezdiatech Inc., Chungcheongnam-do 31116 (KR)
(72) Inventor: JUNG, Yong Gyun, Seoul 02015 (KR); KIM, Ji Yeong, Incheon 22868 (KR); JANG, Gun Hyuk, Seoul 06709 (KR)
(74) Representative: Prüfer & Partner mbB Patentanwälte · Rechtsanwälte
(86) International application number: PCT/KR2021/015522
(87) International publication number: WO 2022/092945

(57) **Abstract**

Provided are microparticle probes for isolating and detecting nucleic acids to detect target nucleic acids. Each of the microparticle probes includes: a microparticle; capture probes introduced on the surface of the microparticle and including sequences complementary to those of the target nucleic acids; and reporter nucleic acids introduced on the surface of the microparticle and generating signals in response to an external stimulus. Also provided are a kit including the microparticle probes, a method for detecting target nucleic acids, and a multiplex diagnostic method. The microparticle probes of the present invention enable rapid identification of multiple viral infections such as respiratory syncytial virus (RSV), influenza, and coronavirus infections and can be used to accurately determine diseases (infectious diseases) with high sensitivity.

## Description

### Technical Field

The present invention relates to microparticle probes for isolating and detecting nucleic acids. More specifically, the present invention relates to microparticle probes for isolating and detecting nucleic acids for multiple diagnostics that enable rapid on-site identification of diseases and can be used to accurately determine diseases with high specificity, a kit including the microparticle probes, a method for detecting target nucleic acids, and a multiplex diagnostic method.

### Background Art

*In vitro* diagnostics (IVD) is a technique for analyzing samples (*e.g*., blood, urine, and cells) collected from humans to diagnose human health. Such *in vitro* diagnostic methods include immunodiagnostics, self-blood glucose monitoring, and molecular diagnostics. Particularly, molecular diagnostics uses molecular biological approaches to diagnose diseases and its applicability has been gradually extended with the discovery of new genes in the fields of genetic and infectious diseases.

Gene editing technology using genetic scissors is a relatively recently developed molecular biological approach to recognize specific nucleotide sequences of genes in cells and edit the nucleotide sequences as desired. Gene editing technology has been spotlighted as an innovative solution that is applicable to gene therapy. Gene editing technology has continuously advanced over generations (First generation: zinc finger nucleases, second generation: transcription activator-like effector nucleases (TALENs), third generation: CRISPR-Cas9). In recent years, efforts to develop new molecular diagnostics using genetic scissors have received as much attention as "gene editing".

COVID-19 is currently spreading around the world. In South Korea, where COVID-19 is relatively well controlled, several dozens of patients with COVID-19 are reported each day. In contrast, other countries are facing serious pandemic situations in which coronavirus causes approximately 300,000 to 400,000 of cases and thousands of deaths each day. However, the diagnosis of COVID-19 is limited in coping with the current situations. Specifically, it takes 1 to 2 days to diagnose COVID-19 in South Korea, where COVID-19 is relatively well controlled. In contrast, 4 to 7 days are usually required to diagnose COVID-19 in the United States, making it impossible to effectively counteract the spread of the disease. In reaction processes for existing molecular diagnostics, partial binding or overlapping of markers induces the amplification of nucleic acids and sequences acting as probes become positive, resulting in the occurrence of false positives. Thus, there is an urgent need for a new on-site diagnostic technology that can produce accurate diagnosis results within 30 to 40 minutes.

Specifically, since the symptoms of COVID-19 are similar to those of influenza and RSV diseases, it is necessary to develop multiple diagnostics for accurately distinguishing three or more different diseases. Particularly, since the development of therapeutic agents for COVID-19, multiple diagnostics is more needed because therapeutic agents for coronavirus (COVID-19), influenza viruses, and RSVs are different from each other.

In connection with the diagnosis of COVID-19, a research team led by Professor Jennifer Doudna at the University of California, Berkeley, USA has recently succeeded in developing diagnostics to determine the presence of coronaviruses using CRISPR gene editing technology, which was awarded the 2020 Nobel Prize in Chemistry. This diagnostic takes a short time for testing and does not require an expensive experimental setup because it avoids the need for viral gene amplification, unlike existing molecular diagnostics. However, there have been no reports that CRISPR gene editing technologies known hitherto are used for multiple diagnostics.

Thus, there is a need for multiple diagnostics that can identify multiple viral infections in a single test even though the symptoms of the infections are not distinct.

### Detailed Description of the Invention

### Problems to be Solved by the Invention

The present invention intends to provide microparticle probes for isolating and detecting nucleic acids that enable rapid identification of multiple viral infections such as respiratory syncytial virus (RSV), influenza, and coronavirus infections and can be used to accurately determine diseases (infectious diseases) with high sensitivity, a kit including the microparticle probes, and a method for detecting target nucleic acids.

### Means for Solving the Problems

According to one aspect of the present invention, there are provided microparticle probes for isolating and detecting nucleic acids to detect target nucleic acids, each of the microparticle probes including: a microparticle; capture probes introduced on the surface of the microparticle and including sequences complementary to those of the target nucleic acids; and reporter nucleic acids introduced on the surface of the microparticle and generating signals in response to an external stimulus.

According to a further aspect of the present invention, there is provided a kit for detecting target nucleic acids, the kit including the microparticle probes for nucleic acid isolation and detection, a restriction enzyme, and a reagent for nucleic acid amplification.

According to another aspect of the present invention, there is provided a method for detecting target nucleic acids, the method including (a) extracting target nucleic acids from a sample, (b) amplifying the target nucleic acids, (c) providing microparticle probes for isolating and detecting the target nucleic acids, each of the microparticle probes including: a microparticle; guide RNAs introduced on the surface of the microparticle and including sequences complementary to those of the target nucleic acids; and reporter nucleic acids introduced on the surface of the microparticle and generating signals in response to an external stimulus, (d) allowing the guide RNAs of the microparticle probes for target nucleic acid isolation and detection to react with the target nucleic acids to form complexes of the microparticle probes for target nucleic acid isolation and detection and the target nucleic acids, (e) allowing a restriction enzyme to bind to the guide RNAs of the microparticle probes for target nucleic acid detection, (f) activating the restriction enzyme to cleave the nucleotide sequences of the target nucleic acids and the reporter nucleic acids, and (g) measuring on/off of signals emitted from the complexes in response to an external stimulus to detect the target nucleic acids.

According to yet another aspect of the present invention, there is provided a multiple diagnostic method for isolating and detecting target nucleic acids in a sample using genetic scissors introduced to the microparticle probes for nucleic acid isolation and detection, the method including: amplifying target nucleic acids in a sample and complementarily binding the target nucleic acids to the microparticle probes for nucleic acid isolation and detection; and allowing genetic scissors introduced to the microparticle probes for nucleic acid isolation and detection to cleave the surrounding reporter nucleic acids when the genetic scissors recognize the complementary binding, wherein a decrease in signal by the cleavage of the reporter nucleic acids is measured to detect the target nucleic acids.

### Effects of the Invention

The microparticle probes for nucleic acid isolation and detection and gene editing technology introduced in the present invention operate to recognize the exact sequences of genes. Therefore, the present invention has increased sensitivity and specificity compared to existing rapid molecular diagnostics (≤ 1 hour) and can accurately diagnose multiple viruses such as coronavirus (COVID-19), influenza viruses, and RSVs, which have similar symptoms, at one time within 30 to 40 minutes, contributing to the maximization of therapeutic effects.

The microparticle probes for nucleic acid isolation and detection according to the present invention can be mixed and moved with high efficiency by using a magnet and can be produced at low cost because they use microparticles with better magnetic properties than conventional magnetic particles (especially magnetic beads). Therefore, the microparticle probes of the present invention can be used at reduced cost for the same diagnosis as existing diagnostic devices using magnetic particles.

The microparticle probes for nucleic acid isolation and detection according to the present invention include different capture probes for simultaneous diagnosis of various biomarkers depending on their type. Therefore, the detection method of the present invention enables multiplex point-of-care test (POCT) diagnostics of target diseases in a short time.

### Brief Description of the Drawings

Fig. 1 shows one embodiment of a microparticle probe for detecting nucleic acids.
Figs. 2 and 3 show a process for detecting target nucleic acids using magnetic microparticles and gene editing technology.

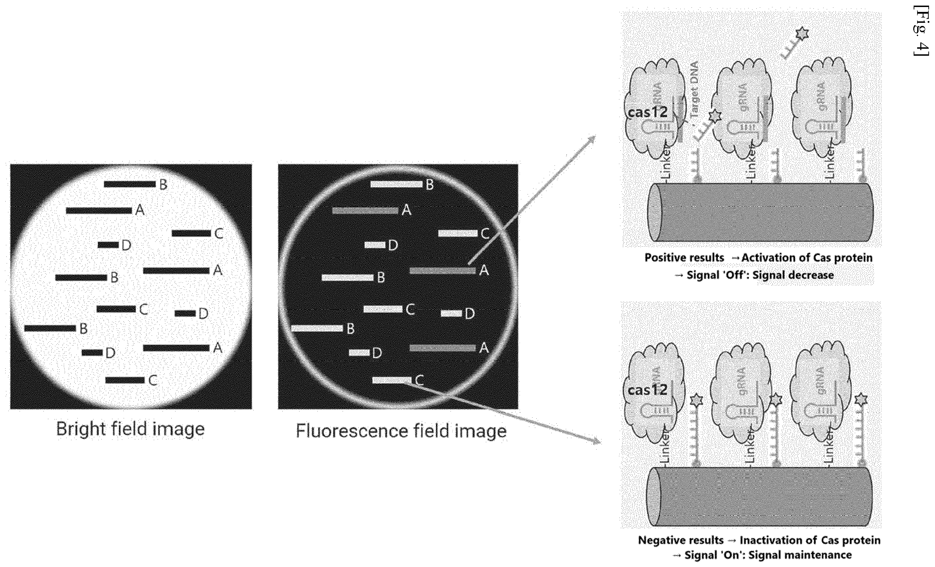

Fig. 4 virtually shows the diagnostic results of the process shown in Figs. 2 and 3.
Fig. 5 shows the results of previously confirming the ability of magnetic particles to capture nucleic acids from target samples.
Fig. 6 shows a process for sequential capture and amplification of target nucleic acids through Well 1 to Well 4.
Fig. 7 shows the results of capture and amplification of RNA and DNA fragments (see SEQ ID NOS: 1 and 2) as nucleic acids.
Fig. 8 shows the results of capture and amplification of nucleic acids from inactivated viruses as targets.
Fig. 9 confirms concentration-dependent changes in the activity of a Cas system by magnetic particles coupled with different concentrations of gRNAs in a state in which free reporter DNAs and a Cas protein were mixed, in the presence of target nucleic acids.
Fig. 10 shows the results of an experiment for confirming the activity of a Cas system for non-target nucleic acids.
Fig. 11 shows the results of a test for coupling of magnetic particles with optimal concentrations of reporter DNAs.
Fig. 12 shows fluorescence signal data from magnetic particles coupled with gRNAs and reporter DNAs in the presence of target nucleic acids.
Fig. 13 shows a change in fluorescence signal from Covid-19-targeted probes in the presence of coronavirus (Covid-19) as a target and a change in fluorescence signal from Covid-19-targeted probes in the presence of influenza A virus or influenza B virus as a target.
Fig. 14 shows a change in fluorescence signal from influenza A-targeted probes in the presence of influenza A virus as a target and a change in fluorescence signal from influenza B-targeted probes in the presence of influenza B virus as a target.
Fig. 15 shows a change in fluorescence signal from a mixture of Covid-19-targeted probes and influenza A-targeted probes in the presence of coronavirus (Covid-19) or influenza A virus as a target and a change in fluorescence signal from a mixture of Covid-19-targeted probes and influenza A-targeted probes in the presence of coronavirus (Covid-19) and influenza A virus as targets.
Fig. 16 shows data for the changes in fluorescence signal analyzed in Fig. 15, which are divided into influenza A probe group and Covid-19 probe group.

### Mode for Carrying out the Invention

It should be understood that the terms and words used in the specification and the claims are not to be construed as having common and dictionary meanings but are construed as having meanings and concepts corresponding to the technical spirit of the present invention in view of the principle that the inventor can define properly the concept of the terms and words in order to describe his/her invention with the best method.

One aspect of the present invention provides microparticle probes for isolating and detecting nucleic acids to detect target nucleic acids.

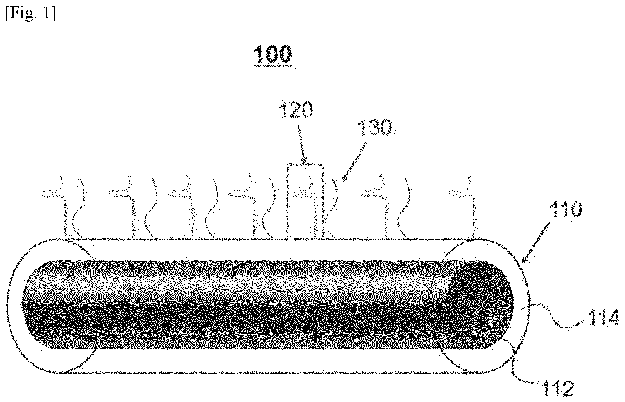

Fig. 1 shows one embodiment of a microparticle probe for detecting nucleic acids.

Referring to Fig. 1, the microparticle probe 100 for nucleic acid isolation and detection includes a microparticle 110, capture probes 120, and reporter nucleic acids 130.

The microparticle 110 offers a surface area for the detection of target nucleic acids. For this purpose, the capture probes 120 and the reporter nucleic acids 130 are introduced on the surface of the microparticle 110. The microparticle 110 may be made of a polymer such as polymethyl methacrylate (PMMA) or polystyrene (PS), an inorganic material such as silica, or a composite thereof. The microparticle 110 may contain magnetic particles to collect detected target nucleic acids or promote the reaction with a reagent.

The microparticle 110 may have a single structure or a core-shell structure. Preferably, the microparticle 110 has a core-shell structure in which a protective shell 114 surrounds a core 112. The core 112 of the microparticle 110 may include a magnetic material, for example, a magnetically responsive metal. The magnetically responsive metal may be a paramagnetic material. Specifically, the magnetically responsive metal may be an iron (Fe), nickel (Ni), cobalt (Co) or manganese (Mn) alloy. The magnetically responsive metal may essentially include at least one transition metal such as iron, nickel, cobalt or manganese and may optionally include at least one rare earth metal such as gadolinium (Gd), terbium (Tb) or samarium (Sm). The magnetically responsive metal may optionally further include one or more other elements such as boron (B), silicon (Si), and carbon (C). The magnetically responsive metal is typically an iron alloy or a cobalt alloy. Specifically, the iron alloy is Fe₇₀B₁₅Si₁₀C₅ and the cobalt alloy is Co₆₈Mn₇Si₁₀B₁₅.

Preferably, the microparticle 110 has a size and specific gravity such that it is not suspended in water. Due to these physical properties, the microparticle 110 can be quickly collected or separated by an external magnet such as a permanent magnet or electromagnet. The core 112 may occupy 60% or more of the total volume of the microparticle 110. For example, the core 112 may occupy 60 to 99%, preferably 75 to 99%, of the total volume of the microparticle 100. Preferably, the microparticle 110 has a size (*e.g.,* a diameter or length) of about several tens to several hundreds of micrometers (µm) and a specific gravity of at least 5. If the microparticle 100 has a size of less than 1 micrometer, it may be suspended in water despite its high specific gravity (for example, 7.876 for an iron nanoparticle). In this case, during a bioassay for detecting biological materials in a well using the microparticle 100 under the influence of an applied magnetic field, the biological materials are difficult to separate because the low magnetism of the microparticle makes control over the magnetic properties of the microparticle difficult. When a magnetic rod is moved up and down in wells to promote immune reactions, microparticles are attached to and detached from the magnetic rod. Even after the magnetic field is removed, the microparticles attached to the magnetic rod do not fall off on the well bottom and remain non-specifically bound to the magnetic rod during upward and downward movement of the magnetic rod due to their small weight, causing poor reproducibility of quantitative analysis when biological materials present in the wells are detected.

According to one embodiment of the present invention, the microparticle probes for nucleic acid isolation and detection are much larger in size than silica beads used for bioassays in the art. The magnetically responsive metal (magnetic core) takes up most of the volume of the microparticles, unlike existing silica beads containing magnetic particles therein. Thus, the magnetically responsive metal is sensitive to magnetism, ensuring high reproducibility of quantitative analysis.

The microparticle probe 100 for nucleic acid isolation and detection has a core-shell structure in which the central magnetically responsive metal is surrounded by the shell layer. The shell layer 114 is formed using an organic or inorganic material, preferably glass. The capture probes are immobilized on the surface of the microparticle 110. The capture probes may be antibodies or proteins. For immobilization of the capture probes, functional groups such as hydroxyl, amino or carboxyl groups may be introduced on the surface of the microparticle.

The shell layer 114 may substantially completely surround the microparticle 110. Alternatively, the surface of the microparticle 110 may not be completely covered with the shell layer and may be partially exposed during production of the microparticle.

The thickness of the shell layer 114 may be 1 to 100 µm, preferably 1 to 50 µm, more preferably 1 to 10 µm, even more preferably 4 to 8 µm. If the thickness of the shell layer 114 is less than the lower limit, the surface of the shell layer 114 tends to be brittle or is apt to crack. Meanwhile, if the thickness of the shell layer 114 exceeds the upper limit, problems may be caused by the characteristics and wavelength of a laser during glass cutting.

The core-shell structure may be formed by applying a liquid shell component to the core metal or filling the core metal component in a hollow frame.

The shell layer 114 may be formed by solidification of an organic or inorganic coating solution. More specifically, the shell layer 114 may be formed by melting an organic or inorganic shell component at a high temperature to make the shell component flowable. Alternatively, the shell layer 114 may be formed by dissolving a shell component in a solvent to prepare a coating liquid and applying the coating liquid to the core metal. The organic material is usually a polymer and the inorganic material may be a metal or a ceramic material, especially glass. For example, the shell layer 114 may be formed by dissolving a plastic material in a solvent or melting glass to obtain a coating solution and applying the coating solution to the core 112 by a suitable coating process such as dip coating or spray coating.

A glass tube may be used as the hollow frame. In this case, after a metal powder is put into the glass tube, the glass tube is drawn while melting the metal powder at high temperature. Alternatively, a molten metal may be injected into the glass tube while drawing the glass material. Alternatively, a dispersion of a glass powder in a UV curable material may be filled in a glass tube and cured by irradiation with ultraviolet light. In these approaches, the hollow frame may constitute the shell layer.

The surface of the microparticle 110 is very uniform. In the case of particles for bioassay, a shell layer 114 is formed by growing a silica precursor such as TEOS on the surface of core particles. In this case, the shell layer 114 has a very rough surface, and as a result, most of the particles bind non-specifically to target materials. This non-specific binding may cause unnecessary background noise.

Glass (for example, borosilicate glass) for the shell layer 114 exhibits little non-specific binding caused by adsorption with a reaction sample through a chemical reaction. Particularly, the surface of the microparticle 110 is very uniform because the shell layer 114 has a coating layer derived from a liquid component. The average surface roughness (Rₐ) of the shell layer 114 may be 15 nm or less, preferably 10 nm or less, more preferably 5 nm or less, more preferably 2 nm or less, particularly 1.5 nm or less. The Rₐ may be, for example, 3 nm or more, 2 nm or more or 1 nm or more. Within this range, non-specific adsorption of a reaction sample to the surface of the shell layer 114 can be minimized.

A material for the shell layer 114 of the microparticle 110 may be glass in terms of strength and transparency. The glass may be composed essentially of at least one compound selected from the group consisting of soda lime, borosilicate, aluminosilicate, silica, alkali silicate, Pyrex, and quartz. Preferably, the glass is borosilicate glass, which is suitable for experiments where heat resistance, acid resistance, and water resistance are required.

The microparticles 110 may be in an unshaped or shaped form. When shaped, the microparticles 110 are in the form of rods, sheets or spheres. The sheet-like microparticles may have various cross-sectional shapes such as stars, polygons, and circles but are not particularly limited thereto. The microparticles are in the form of microrods, microdiscs or microbeads, which are preferable in terms of convenience of production and ease of observation. The microparticles are particularly preferably in the form of microrods. The microparticles in the form of microrods are easy to distinguish from each other due to their overlap. When placed in wells, the microparticles in the form of microrods are easy to focus on. Since the individual particles in the form of microrods occupy small areas, a large number of the microparticles can be displayed on a single screen. The microparticles may have complex cross-sectional shapes such as stars. In this case, however, the microparticles tend to collide with each other or with the walls of the wells while moving in the wells, causing breakage at the edges. Therefore, it is advantageous that the microparticles have simple shapes such as microrods.

The microrods may have a length of 10 to 1,000 µm. If the microrods have different lengths less than the lower limit, they are not easy to distinguish from each other. Meanwhile, if the length of the microrods exceeds the upper limit, the particles may overlap, making their observation difficult. The ratio of the length to the diameter (i.e. aspect ratio) of the microrods may be at least 2, at least 5 or at least 10 and may be 20 or less. If the aspect ratio is less than 2, the microrods are close to spherical microparticles, making it difficult to distinguish from each other. Meanwhile, if the aspect ratio exceeds 20, the microrods are liable to bend.

In a preferred embodiment, the microparticles 110 may be obtained by cutting glass-coated metal microwires. In this embodiment, the glass-coated metal microwires are simply cut to different lengths by a laser.

The microparticles can be suitably used for *in vitro* diagnostics for detecting biological materials in samples derived from living organisms. The samples may be tissue extracts, cell lysates, whole blood, plasma, serum, saliva, ocular humor, cerebrospinal fluid, sweat, urine, milk, ascites fluid, synovial fluid, and peritoneal fluid. For rapid diagnosis, the pretreatment of the samples may be simplified or may be even omitted.

The microparticles may be designed to have different sizes, lengths or shapes for simultaneous diagnosis of a plurality of biomarkers present in a sample derived from a living organism.

The capture probes 120 may be introduced onto the microparticle 110 to capture biomarkers (particularly nucleic acid-based biomarkers) derived from a sample. The biomarkers are not limited as long as they are used in general scientific or medical applications for the measurement or evaluation of biological treatment, pathogenesis, and pharmacological treatment processes. The biomarkers may be, for example, polypeptides, peptides, nucleic acids, proteins or metabolites that can be detected in biological fluids such as blood, saliva, and urine. The biomarkers are preferably nucleic acids that are associated with specific diseases and can be detected with high sensitivity and specificity.

According to one embodiment of the present invention, the capture probes 120 may be introduced onto the shell layer 114 of the microparticle probe to capture target nucleic acids extracted from a sample. The capture probes 120 complementary to target nucleic acids specifically bind to the target nucleic acids. Thus, the capture probes 120 serve to immobilize the target nucleic acids onto the microparticle 110.

The capture probes 120 may include nucleotide sequences for the application of gene editing technology. Preferably, the capture probes 120 are or include guide RNAs (gRNAs). The guide RNAs refer to RNAs specific to target DNAs (e.g., RNAs capable of hybridizing with target sites of DNAs). The guide RNAs together with restriction enzyme may be used as elements of a CRISPR system. Each of the guide RNAs includes two smaller guide RNAs, that is, a CRISPR RNA (crRNA) having a nucleotide sequence capable of hybridizing with a target site of a gene and an additional trans-activating crRNA. Each of the guide RNAs may be in the form of a crRNA:tracrRNA complex (dual guide RNA) in which the crRNA is partially bound to the tracrRNA or a single guide RNA (sgRNA) in which the crRNA (a portion or the entirety thereof) is linked to the tracrRNA (a portion or the entirety thereof) via a linker. The gRNAs bind to the target nucleic acids and can cleave the target nucleic acids and the reporter nucleic acids when they encounter a restriction enzyme. Based on such characteristics, the guide RNAs can cleave the sequences of specific biomarkers, and as a result, a change in detection signal can be obtained, as will be described later. Information on the change in detection signal can be used to rapidly detect the biomarkers.

Specifically, the capture probes 120 may be 15- to 60-mer nucleic acids. The nucleic acids may be 20- to 45-mer, 25- to 40-mer, or 30- to 41-mer in length.

The capture probes 120 may be immobilized on the surface of the microparticle 110 by adsorption or chemical linkage by adsorption or chemical linkage. Preferably, the capture probes 120 are linked to the surface functional groups of the shell layer 114. For example, the capture probes 114 may be attached to the microparticle 110 by biotinylating the surface of the capture probes 120 and introducing biotin-binding avidin, neutravidin or streptavidin to the microparticle 110. Alternatively, the capture probes 120 may be linked to the microparticle 110 via hydroxyl, amino or carboxyl groups on the surface of the microparticle 110.

The use of the microparticle probes for nucleic acid isolation and detection enables quick analysis desired biological materials in sample solutions with high sensitivity.

The reporter nucleic acids 130 together with the capture probes 120 are introduced on the surface of the microparticle 110 and generate signals, for example, optical or electrical signals, in response to an external stimulus such as chemical, mechanical, optical or electrical energy. In one embodiment, the reporter nucleic acids 130 may be labeled with a luminescent material and may generate luminescence signals such as fluorescence or chemiluminescence signals in response to external light or a chemical reaction.

Specifically, the reporter nucleic acids 130 may be DNAs. The reporter nucleic acids may be 15- to 40-mer in length. The reporter nucleic acids may be 18- to 30-mer, 19- to 25-mer or 20- to 23-mer in length.

The luminescent material is bound to the detection probes and generates light in response to an external stimulus to detect the presence of target nucleic acids. The external stimulus can be selected from ultraviolet light, electron beams, chemical reactions, and enzyme-substrate reactions. Examples of suitable luminescent materials include fluorescent molecules, quantum dots, metal nanoparticles, magnetic nanoparticles, enzymes, and enzyme substrates. Particularly, fluorescent molecules are preferred because of their ease of purchase and convenience of application. The fluorescent molecules can be selected from the group consisting of fluorescein isothiocyanate (FITC), fluorescein, fluorescein amidite (FAM), phycoerythrin (PE), tetramethyl-rhodamine isothiocyanate (TRITC), cyanine 3 (Cy3), cyanine 5 (Cy5), cyanine 7 (Cy7), Alexa Fluor dyes, and rhodamine.

For the detection of various biomarkers in a sample, various fluorophores such as FITC, PE, and Alexa-647 may be introduced at the 5' ends of the reporter nucleic acids 130 for labeling.

According to one embodiment of the present invention, the microparticle probes for target nucleic acid isolation and detection may be applied to rapid and accurate detection of target nucleic acids when a gene editing system is introduced.

The reporter nucleic acids 130 may be nucleotide sequences to be cleaved when the microparticle probes for nucleic acid detection are utilized in a gene editing system. According to gene editing technology, DNA insertion, deletion, and substitution in specific genomic regions are induced using protein complexes having a DNA nuclease activity to cause mutations. Gene editing technology includes zinc finger nucleases (ZFNs), transcription activator-like effector nucleases (TALENs), and CRISPR-Cas systems. Gene editing technology is preferably a CRISPR-Cas system that has an outstanding ability to accurately cleave desired DNA sites in a simple and fast manner. Clustered regularly interspaced short palindromic repeats (CRISPRs) act as guide RNAs (gRNAs) to recognize target DNAs and form complexes with a restriction enzyme such as a Cas endonuclease to cleave DNA double strands. Any CRISPR-Cas system known in the art may be used without any particular limitation in the present invention. CRISPR-Cas systems are classified into CRISPR-Cas9, CRISPR-Cas12, and CRISPR-Cas13 depending on the type of the restriction enzyme. The CRISPR-Cas system is preferably CRISPR-Cas12 that has high sensitivity to mismatches within guide RNAs, more preferably CRISPR-Cas 12a using Cas12a protein as a restriction enzyme.

In one embodiment, the reporter nucleic acids 130 may be attached to the surface of the microparticle 110 at a high density for reaction with a Cas endonuclease, which is to be introduced later as a protein complex functioning as a DNA nuclease. The reporter nucleic acids 130 may be introduced on the surface of the microparticle 110, for example, at a density of 5×10¹⁶ counts/m² to 10×10¹⁶ counts/m², preferably 6×10¹⁶ counts/m² to 7×10¹⁶ counts/m². In this case, a strong signal may be generated to easily distinguish the size and shape of the microparticle 110. The reporter nucleic acids 130 may have single- or double-stranded sequences depending on the type of the Cas endonuclease. In one embodiment, the Cas endonuclease operates according to a mechanism in which it is complexed with conjugates of the microparticle probe 100 (that is, in a state in which the capture probes 120 and the reporter nucleic acids 130 are uniformly distributed on the surface of the microparticle 110) and target nucleic acids and cleaves the reporter nucleic acids 130 distributed around the conjugates of the capture probes 120 and the target nucleic acids.

When amplification products of target nucleic acids obtained by pretreatment of a sample collected from a patient are allowed to react with the microparticle probes 100 for target nucleic acid isolation and detection, specific target nucleic acids recognizing the amplified single-stranded regions may complementarily bind to the gRNAs of the capture probes to form complexes. When a Cas endonuclease such as Cas12a protein used in a CRISPR system is applied to the complexes, the target nucleic acids bind to the spacer sequences of the guide RNAs to activate the sequence cleavage activity of Cas12a protein, resulting in cleavage of the target nucleic acids and the surrounding reporter nucleic acids. As a result, a phenomenon is observed in which fluorescence signals are not maintained and disappear.

Thus, bright field images and fluorescence field images of the magnetic microparticle probes are simultaneously obtained before and after the application of a Cas endonuclease and compared with each other. As a result, disappearance of fluorescence from the microparticles can be observed and the presence of specific nucleic acids in the sample can be determined. In conclusion, when the gene editing system is applied, the use of microparticles having various shapes or sizes and including various types of phosphors enables multiple diagnostics.

A further aspect of the present invention provides a kit for detecting target nucleic acids.

The kit includes the microparticle probes for target nucleic acid isolation and detection and a restriction enzyme. The microparticle probes have been described in detail above. A Cas endonuclease is a preferred example of the restriction enzyme. The Cas endonuclease forms complexes with the guide RNAs and acts as a component of a CRISPR-Cas system to cleave the reporter nucleic acids. The Cas endonuclease may be, for example, Cas9 or Cas 12a protein.

In one embodiment, the kit may further include one or more components selected from the group consisting of magnetic microparticles for capturing target nucleic acids, reagents for amplifying nucleic acids, lysis solutions, and cleaning solutions.

The magnetic microparticles for target nucleic acid capture are constructed to have the characteristics of silica on the surface thereof. This construction is effective in increasing the area of the surface and forming salt bridges in the presence of a high concentration of a chaotropic salt in a lysis solution, facilitating the capture of nucleic acids. The magnetic microparticles may be silica magnetic beads or silica-coated magnetic microparticles. The reagent for nucleic acid amplification is not particularly limited as long as it can amplify target nucleic acids. Examples of suitable reagents for nucleic acid amplification include loop-mediated isothermal amplification (LAMP), where nucleic acid amplification is performed under isothermal conditions, single primer isothermal amplification (SPIA), and recombinase polymerase amplification (RPA) reagents. An RPA reagent is preferable in terms of speed and low-temperature reactivity. For example, the RT-RPA reagent may contain target-specific primers for specifically amplifying RSV, influenza, and coronavirus genes. The lysis solution may be a sample lysis solution based on a chaotropic salt such as a guanidinium salt. The cleaning solution may be an ethanol-based solution for sample cleaning.

The kit for target nucleic acid detection may be combined with an integrated device for driving the kit for nucleic acid detection to constitute an overall system for detecting nucleic acids. The integrated device is provided with a magnetic rod for sample separation and reaction promotion. The magnetic rod allows the integrated device to smoothly drive the magnetic microparticles for nucleic acid capture and the microparticle probes 100 for signal detection in a lysis sample.

In the kit for target nucleic acid detection, starting from the spreading of a sample collected irrespective of the type of the sample, many reactions proceed in a fully automated process. Such reactions include lysis of the sample, nucleic acid capture and amplification, capture of the amplified target nucleic acids, and Cas12a activation due to the captured target nucleic acids. The absence or presence of target substances can be rapidly and accurately detected by measuring a decrease in fluorescence signal due to the recognized target nucleic acids.

Another aspect of the present invention provides a method for detecting target nucleic acids.

First, (a) target nucleic acids are extracted from a sample. A lysis solution containing guanidinium thiocyanate may be used to disrupt the cell membrane of the sample and liberate nucleic acids from the cells. The nucleic acids in the solution may be attached to magnetic silica beads or the silica-coated microparticles 110 of the present invention. The attached nucleic acids are concentrated, washed with alcohol, and detached using an elution buffer.

Then, (b) the target nucleic acids are amplified. For example, the target nucleic acids may be specifically amplified using a reverse transcription real-time recombinase polymerase amplification (RT-RPA) reagent.

Subsequently, (c) microparticle probes for isolating and detecting the target nucleic acids are provided. Each of the microparticle probes for target nucleic acid isolation and detection includes: a microparticle; guide RNAs introduced on the surface of the microparticle and including sequences complementary to those of the target nucleic acids; and reporter nucleic acids introduced on the surface of the microparticle and generating signals in response to an external stimulus. Details of the components of the microparticle probes are the same as those described above.
(d) The guide RNAs of the microparticle probes for target nucleic acid isolation and detection are allowed to hybridize with the target nucleic acids to form complexes of the microparticle probes for target nucleic acid isolation and detection and the nucleic acids.

In one embodiment, the guide RNAs immobilized onto the microparticles for target nucleic acid isolation and detection may target only specific viral species and the reporter DNAs immobilized onto the microparticles for target nucleic acid isolation and detection may be attached with 5'-fluorophores. The gRNAs of the specific targeted magnetic particle probes can recognize single-stranded regions to be amplified and can complementarily bind with viral genes to form complexes. The formation of the complexes may include promoting the reactions in the presence of an external magnetic force. For example, a well containing the reactants may be heated to an appropriate temperature to promote the reactions. A magnetic rod is continuously moved up and down in the well to control the microparticles containing a magnetic material such that the reactions are promoted.
(e) A restriction enzyme is allowed to bind to the guide RNAs of the microparticle probes for target nucleic acid isolation and detection. A preferred example of the restriction enzyme is a Cas endonuclease, for example, Cas9 or Cas12a.

The restriction enzyme recognizes and binds to the scaffold sequences of the gRNAs immobilized onto the microparticles for target nucleic acid isolation and detection.

Thereafter, (f) the restriction enzyme is activated to cleave the nucleotide sequences of the target nucleic acids and the reporter nucleic acids. The Cas protein is activated in a state in which the amplified target nucleic acids are bound to the gRNAs, resulting in the cleavage of the nucleotide sequences of the target nucleic acids and the reporter nucleic acids.

After completion of the reactions, the microparticle probes may be washed with a washing buffer. Preferably, the method includes washing the microparticle probes in the presence of an external magnetic force. That is, the method may further include continuously washing the microparticle probes for target nucleic acid detection using magnetic rods in two or more washing wells after the reactions are completed. Luminescence signals from the microparticle probes can be detected after the washing to accurately identify the types and amounts of the target nucleic acids.
(g) On/off of signals emitted from the complexes in response to an external stimulus is measured to detect the target nucleic acids. The signals may be luminescence signals emitted from the luminescent material in the complexes. The external stimulus can be selected from ultraviolet light, electron beams, chemical reactions, and enzyme-substrate reactions. For example, before treatment with the Cas endonuclease, all microparticle probes may generate fluorescence ("on" state) by the fluorescent molecules bound to the reporter nucleic acids in the presence of an external stimulus such as ultraviolet light. Thereafter, the nucleotide sequences of the reporter nucleic acids together with the target nucleic acids are cleaved by the Cas endonuclease bound to the guide RNAs. As a result of the cleavage, fluorescence from the microparticles attached with the target nucleic acids is turned "off". After a fluorescence field image and a bright field image of the microparticle probes are merged with each other, the microparticle probes complexed with the target nucleic acids can be specified by analyzing the images to distinguish the target nucleic acids.

Yet another aspect of the present invention provides a multiple diagnostic method for isolating and detecting target nucleic acids in a sample using genetic scissors introduced to the microparticle probes for nucleic acid isolation and detection. Specifically, the method includes: amplifying target nucleic acids in a sample and complementarily binding the target nucleic acids to the microparticle probes for nucleic acid isolation and detection; and allowing genetic scissors introduced to the microparticle probes for nucleic acid isolation and detection to cleave the surrounding reporter nucleic acids when the genetic scissors recognize the complementary binding. The genetic scissors may be a CRISPR-Cas system. In this case, the target nucleic acids can be detected by measuring a decrease in signal by the cleavage of the reporter nucleic acids. The signal may be fluorescence or chemiluminescence.

The two or more types of microparticle probes for nucleic acid isolation and detection may be labeled with length, diameter, thickness, shape, color or identification codes so as to be distinguished from each other. The microparticle probes for nucleic acid detection may have different lengths, sizes or shapes and may include different capture probes.

The multiple detection may include reading the microparticle probes labeled in various ways. For example, the sample may include a plurality of nucleic acid biomarkers and the microparticle probes may have a plurality of shapes whose number corresponds to that of the biomarkers. In this case, capture probes specifically binding to and capturing only specific ones from a plurality of nucleic acid biomarkers present in the sample are immobilized onto the microparticles having different shapes. This enables simultaneous detection of a plurality of biomarkers. The microparticle probes may be, for example, microrods. When microrods having different lengths and gene editing technology such as a CRISPR-Cas system are used, the types and amounts of biomarkers as target nucleic acids can be quickly determined in an independent manner by analyzing a bright field image and a fluorescence field image of the microparticle probes. For example, when microrods produced by cutting glass-coated microwires to various lengths are used as the microparticle probes, multiple detection is enabled with only one fluorescent material. When the microparticle probes coded with length information are analyzed through image analysis, multi-detection is enabled at a time while maintaining highly sensitive accuracy.

That is, the use of two or more types of microparticle probes for nucleic acid isolation and detection enables multiple diagnostics through a single detection process for various target nucleic acids.

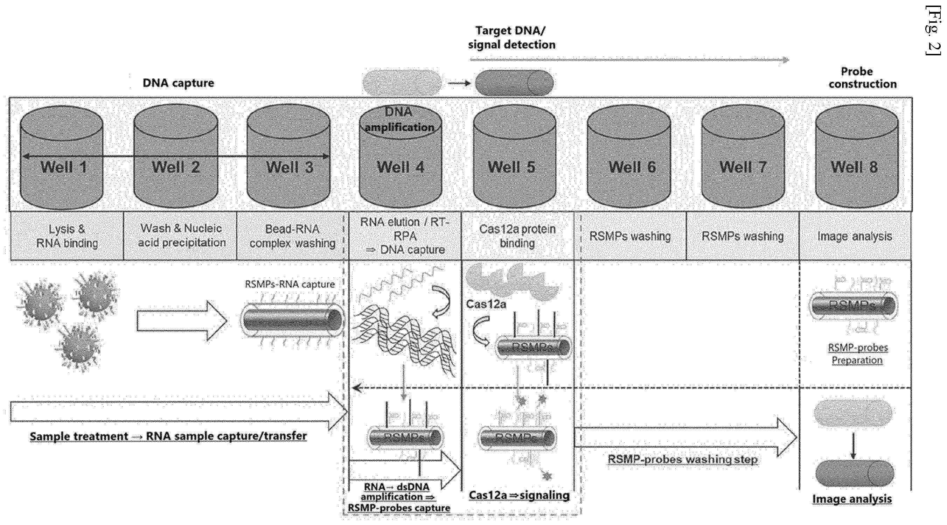

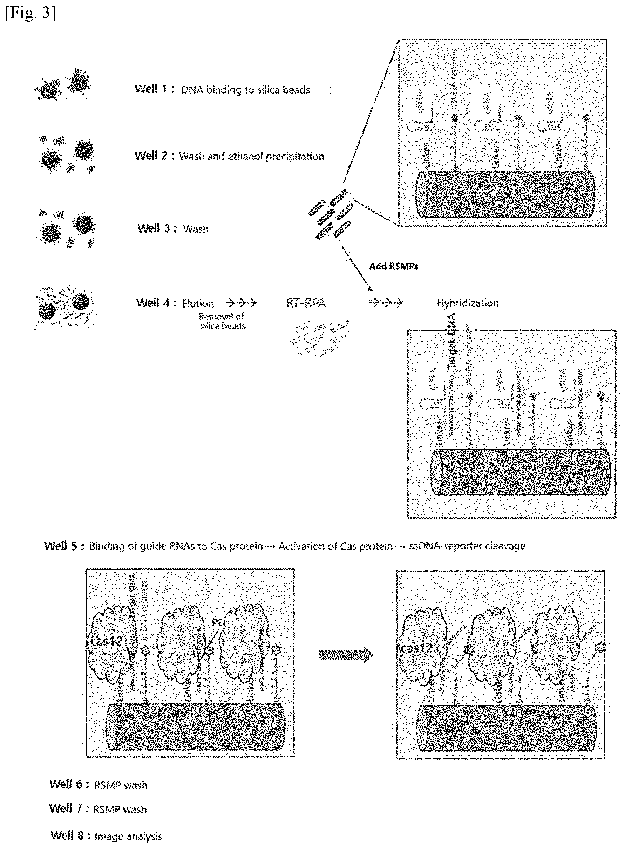

Figs. 2 and 3 show a process for detecting target nucleic acids using magnetic microparticles and gene editing technology. Referring to Figs. 2 and 3, the process is implemented in eight wells (Well 1 to Well 8). Specifically, DNAs are captured in Well 1 to Well 3, the DNAs are amplified and hybridized in Well 4, and images are analyzed in Well 5 to Well 8. The procedure in each well will be described in more detail below.

### 1) Well 1: Binding of DNAs to silica beads

A sample is put into Well 1 containing magnetic silica beads, a chaotropic salt, and a lysis solution. The cell membrane of the sample is disrupted and nucleic acids are liberated from the cells. The nucleic acids are attached to the magnetic silica beads by the action of the chaotropic salt.

### 2) Well 2: Washing and precipitation with ethanol

The magnetic silica beads attached with the nucleic acids are transferred to Well 2. 33% isopropanol is used to improve the reactions between the DNAs and the salt. As a result, the DNAs are strongly attached to the magnetic silica beads and unnecessary residues are removed.

### 3) Well 3: Washing

The magnetic silica beads bound with the nucleic acids are washed with 70% ethanol. As a result, residues other than the nucleic acids are further removed as much as possible.

### 4) Well 4: Amplification and hybridization of the nucleic acids

The magnetic silica beads bound with the nucleic acids are transferred to Well 4 containing an elution buffer to detach the nucleic acids therefrom. Then, cDNAs are synthesized and amplified with an RT-RPA reagent present in Well 4. The RT-RPA reagent may include target-specific primers for specifically amplifying RSV, influenza virus, and coronavirus genes.

Three types of magnetic microparticle probes having different lengths are prepared. Each of the magnetic microparticle probes having specific lengths is immobilized guide RNAs targeting only specific viral species and reporter DNAs in the form of single-stranded random DNAs and attached with 5'-fluorophores for signal detection. As the viral genes are amplified by RT-RPA, the gRNAs of the specific targeted magnetic particle probes recognizing the amplified single-stranded regions complementarily bind with the viral genes to form complexes of the magnetic microparticle probes and the viral gene sequences.

### 5) Well 5: Binding of Cas protein and cleavage of the reporter DNA sequences

After completion of the reactions in Well 4, the magnetic microparticle probes in the form of round-shaped magnetic particles (RSMPs) are transferred to Well 5 containing Cas12a endonuclease. The Cas12a protein recognizes and binds to the scaffold sequences of the gRNAs immobilized onto the magnetic particle probes. When gRNA-cas12a protein complexes are formed and the target DNAs are amplified and bound to the spacer sequences of the gRNAs in Well 4, the endonuclease activity of the Cas12a protein is activated, resulting in cleavage of the target DNAs and the surrounding non-specific DNAs. In contrast, when the target DNAs are not amplified in Well 4, gRNA-Cas12a protein complexes are formed in Well 5 but the target DNAs are not bound to the spacer sequences of the guide RNAs, failing to activate the endonuclease activity of the Cas12a protein.

### 6) Well 6: Washing

After the reactions are completed, the magnetic microparticle probes are washed with a washing buffer.

### 7) Well 7: Washing

After the reactions are completed, the magnetic microparticle probes are washed once more with a washing buffer.

### 8) Well 8: Image analysis

After completion of the reactions and washing, the magnetic microparticle probes are imaged. Specifically, both a bright field image and a fluorescence field image of the magnetic microparticle probes are obtained.

The microparticle probes for nucleic acid isolation and detection and the multiple diagnostic method using the microparticle probes have the following advantages. The present invention uses CRISPR-CAS gene editing technology for the development of an emergency diagnostic platform for respiratory viruses to overcome the limitations of existing molecular diagnostics based on reverse transcriptase-polymerase chain reaction (RT-PCR). Existing molecular diagnostic methods include on-site sampling and gene extraction, amplification, and detection at a depository institution and use expensive systems to obtain the results of gene amplification. Such processes are not suitable for in-situ large-scale sample inspection and take at least 6 hours or more to obtain the results. In contrast, the present invention uses CRISPR-Cas gene editing technology to enable in situ gene extraction, amplification, and detection in an automated manner (the use of magnetic particles can greatly reduce the time for gene extraction and amplification). In addition, the use of CRISPR-Cas gene editing technology in the present invention overcomes the phenomenon of non-specific amplification found in existing molecular diagnostics.

The microparticle probes for nucleic acid isolation and detection and gene editing technology introduced in the present invention operate to recognize the exact sequences of genes. Therefore, the present invention has increased sensitivity and specificity compared to existing molecular diagnostics and can diagnose multiple viruses such as coronavirus (COVID-19), influenza viruses, and RSVs within 30 to 40 minutes. Therefore, the present invention enables accurate multiple diagnostics at one time, achieving maximal therapeutic effects. Since the detection method of the present invention enables simultaneous diagnosis of various biomarkers, its use is suitable for multiplex point-of-care test (POCT) diagnostics.

The present invention will be explained in more detail with reference to the following examples. However, it will be obvious to those skilled in the art that these examples are provided for illustrative purposes only and do not serve to limit the scope and spirit of the invention.

### [EXAMPLES]

In the following examples, experimental steps were arbitrarily divided into target factor lysis and nucleic acid capture/amplification, preparation of magnetic particle probes for signal detection, detection of signals from the target factor reactions, etc., which were performed in a single process. The individual steps are described in detail below.

### Example 1. Preparation of magnetic microparticles for nucleic acid capture

Eight wells were used in this multiple diagnostic system. For the transfer of nucleic acids between the individual wells, it was essential to use magnetic microparticles that can stably capture nucleic acids extracted from a target factor and move the nucleic acids between the wells. For stable capture of the nucleic acids, magnetic particles for nucleic acid capture having a sufficient surface area were prepared from cylindrical magnetic microparticles or commercially available magnetic microparticles having a size of 10-1000 µm as raw materials.

First, home-made standard magnetic microparticles corresponding to 110 in Fig. 1, which had a core-shell structure without capture probes and were 100-500 µm in length and cylindrical rod-like in shape, were washed three times with a 0.1 M sodium hydroxide solution, washed three times with a 100% ethanol solution, and cleaned by ultrasonic washing for 2 sec.

Then, 200 mg of the magnetic microparticles were placed in 10 ml of a solution containing 20 ml of 100% ethanol, 3 ml of deionized water, and 1 ml of aqueous ammonia, and 2 ml of a tetraethyl orthosilicate (TEOS) solution was added thereto (TEOS) such that the amount of TEOS was 100 µl per 10 mg of the magnetic particles. The resulting solution was rotated at 20 rpm for 20 min using a rotator (FINEPCR, Hybridization Incubator Combi-H12), which was defined as one cycle of reaction. A total of 4 cycles of reaction were carried out to complete the preparation of the magnetic microparticles. 2 ml of a TEOS solution was added in each cycle of reaction. The resulting magnetic particles were washed three times with the ammonia solution prepared above and three times with 100% ethanol before storage in an ethanol solution.

The abilities of the magnetic microparticles to capture nucleic acids from samples were confirmed. Viral or harmful bacterial species may be presumed as the samples. Here, *E. coli* samples were selected and the abilities of the magnetic microparticles to capture nucleic acids from the *E. coli* samples were confirmed.

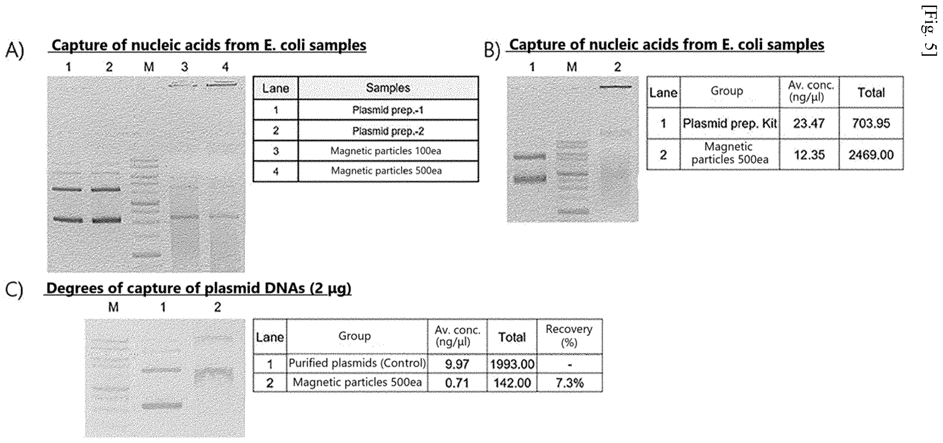

Fig. 5 shows the results of previously confirming the ability of the magnetic particles to capture nucleic acids from the target samples. A) of Fig. 5 compares the results of purification and isolation of plasmid DNAs from the *E. coli* samples as target factors based on commercial *E. coli* plasmid mini prep. kits (Bioneer, AccuPrep^{®} Plasmid Mini Extraction Kit) and the results of purification and isolation of plasmid DNAs from the *E*. *coli* samples by agarose gel electrophoresis through magnetic microparticle beads for nucleic acid capture prepared based on the home-made magnetic microparticles.

Referring to A) of Fig. 5, plasmid DNAs were purified and captured by the home-made magnetic particles for nucleic acid purification. Here, the numbers of the home-made magnetic particles were 100 and 500.

Lysis buffer: 1 ml of a concentrated sample of *E. coli* cultured in a buffer in Well 1 was mixed with the home-made magnetic microparticle beads and then the mixture was stirred with rotation for 10 min. The buffer was composed of 2 M guanidinium thiocyanate, 36.7 mM Tris-Cl, 16.7 mM EDTA, 2% Triton X-100, 33% isopropanol, and RNase free water. Lanes 1, 2: results of purification using commercial plasmid prep. kits, lane 3: results of purification using the home-made beads (100 ea) for nucleic acid purification, lane 4: results of purification using the home-made beads (500 ea) for nucleic acid purification, and lane M: 1 kb DNA ladder (Solgent, 1 Kb Plus DNA Ladder).

B) of Fig. 5 confirms the results of purification of nucleic acids using plasmid prep. kits and the home-made beads (500 ea) for nucleic acid purification under the same conditions as those shown in A) of Fig. 5. Referring to B) of Fig. 5, the results of nucleic acid purification show that the home-made magnetic particles can purify and recover plasmid DNAs as well as genomic DNAs of *E. coli* compared to the plasmid prep. kits.

C) of Fig. 5 confirms the abilities of the home-made magnetic particles to capture 2 µg of target plasmid DNAs. Referring to C) of Fig. 5, the home-made magnetic particles for nucleic acid purification captured about 7.3% of the nucleic acids from the sample, demonstrating the ability of the home-made magnetic particles to capture nucleic acids.

### Example 2. Target factor lysis and nucleic acid capture/amplification (Well 1-Well 4

An investigation was made as to the applicability of the home-built automated system in which all procedures from lysis of the target sample to imaging of the magnetic particle probes were performed in a single process. A mobility test was conducted to confirm whether target nucleic acids obtained by lysis and capture were normally moved between wells and the process was carried out sequentially.

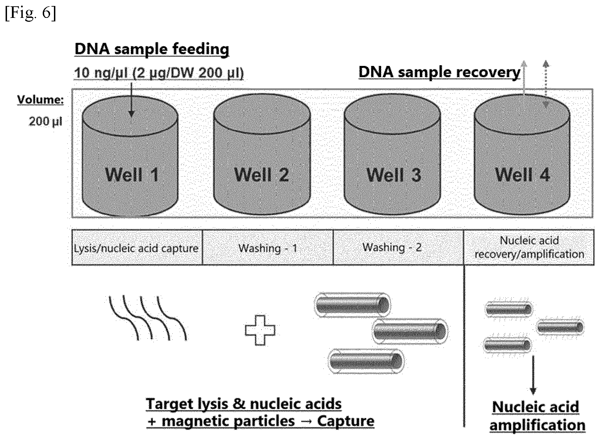

Fig. 6 confirms the capture of nucleic acids from a target sample and the amplification of the target nucleic acids in a multiple diagnostic method according to one embodiment of the present invention.

Fig. 6 shows a process for sequential capture and amplification of target nucleic acids through Well 1 to Well 4. Referring to Fig. 6, lysis was performed and nucleic acids were captured in Well 1. Nucleic acids were extracted and captured from a virus sample using a solution containing a chaotropic salt (2 M guanidinium thiocyanate, 36.7 mM Tris-Cl, 16.7 mM EDTA, 2% Triton X-100, 33% isopropanol, and RNase free water).

A first washing step (washing-1) was carried out in Well 2. Specifically, the magnetic particles including the captured nucleic acids were washed with 2 M guanidinium thiocyanate, 36.7 mM Tris-Cl, 16.7 mM EDTA, 2% Triton X-100, RNase free water, and 66% isopropanol and the captured nucleic acids were further condensed into the magnetic particles.

A second washing step (washing-2) was carried out in Well 3. Specifically, residual waste attached to the magnetic particles was removed using 70% ethanol.

The nucleic acids were recovered and amplified in Well 4. Specifically, the nucleic acids attached to the magnetic particles were detached and amplified. A solution based on recombinase polymerase amplification (RPA) enzyme complexes was used to amplify the nucleic acids.

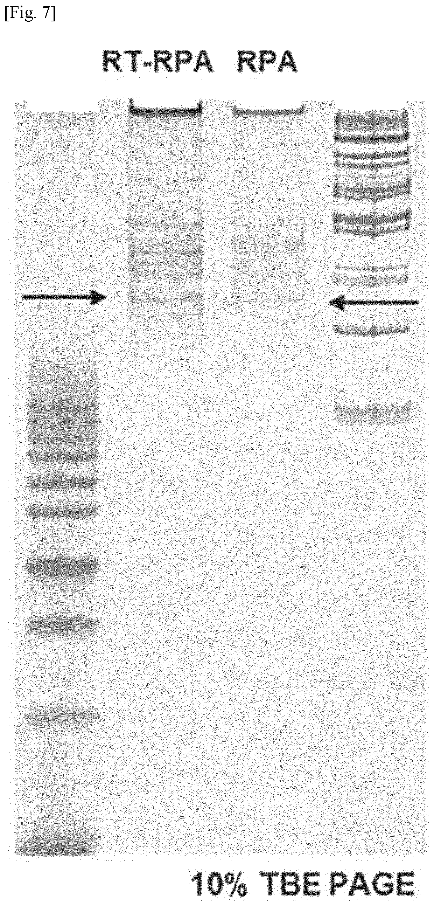

Fig. 7 shows the results of capture and amplification of RNA and DNA fragments (see SEQ ID NOS: 1 and 2) as nucleic acids. The target nucleic acids detached in Well 4 were subjected to reverse transcriptase recombinase polymerase amplification (RT-RPA) and RPA at 37 °C for 20 min in the presence of arbitrary RNA or DNA fragments. Each reaction was carried out using an RPA reagent according to the protocol recommended by the manufacturer (TwistDx, TwistAmp Liquid Kit). Referring to Fig. 7, after each of the RNA and DNA samples was captured and moved between the wells, the nucleic acids were dissociated in Well 4 and were then normally amplified by RT-RPA and RPA enzyme complexes. TBE PAGE gel electrophoresis was performed to more clearly determine the sizes of amplified nucleic acids smaller than 700 bases.

The same test was conducted on inactivated influenza A virus, influenza B virus, and coronavirus (Covid-19) as targets. Table 1 shows information on the inactivated viruses. Tables 2 to 6 are lists of the nucleotide sequences of nucleic acids used.

**[Table 1]**

| **Covid-19 (SARS-CoV-2/USA-WA1/2020, Culture Fluid, Heat Inactivated)** |
|---|
| ZeptoMetrix, Cat. No. 0810587CFHI |

| **Influenza A virus (California/07/09(H1N1), Culture Fluid, Heat Inactivated)** |
|---|
| ZeptoMetrix, Cat. No. 0810165CFHI |

| **Influenza B virus (Florida/02/06, Culture Fluid, Heat Inactivated)** |
|---|
| ZeptoMetrix, Cat. No. 0810037CFHI |

**[Table 2]**

| SEQ ID NOS: 1 and 2: Information on the arbitrary target nucleic acids (RNA/DNA) |
|---|
| Covid-19 (GenBank: MW811435.1, SARS-CoV-2/human/USA/USA-WA1/2020, N gene) |
| RNA: Synthesis of artificial RNA through T7 promoter |
| GGGCGAAUUGGGUACCAACACAAGCUUUCGGCAGACGUGGUCCAGAACAAACCCAAGGA AAUUUUGGGGACCAGGAACUAAUCAGACAAGGAACUGAUUACAAACAUUGGCCGCAAAU UGCACAAUUUGCCCCCAGCGCUUCAGCGUUCUUCGGAAUGUCGCGCAUUGGCAUGGAAGU CACACCUUCGGGAACGUGGUUGACCUACACAGGUGCCAUCAAAUUGGAUGACAAAGAUCC AAAUUUCAAGCUUGAUAUCGAAUUCCUGCAGCCCGGGGGAUCCACUAGUU (SEQ ID NO: 1) |
| DNA: Preparation of DNA sequence through KpnI/Xbal restriction enzymes |
| CAACACAAGCTTTCGGCAGACGTGGTCCAGAACAAACCCAAGGAAATTTTGGGGACCAGGA ACTAATCAGACAAGGAACTGATTACAAACATTGGCCGCAAATTGCACAATTTGCCCCCAGC GCTTCAGCGTTCTTCGGAATGTCGCGCATTGGCATGGAAGTCACACCTTCGGGAACGTGGTT GACCTACACAGGTGCCATCAAATTGGATGACAAAGATCCAAATTTCAAGCTTGATATCGAAT TCCTGCAGCCCGGGGGATCCACTAGTT (SEQ ID NO: 2) |

**[Table 3]**

| SEQ ID NOS: 3 to 5: Information on target sequences of the target samples |
|---|
| Covid-19 (GenBank: MW811435.1, SARS-CoV-2/human/USA/USA-WA1/2020, N gene) |
| AACACAAGCTTTCGGCAGACGTGGTCCAGAACAAACCCAAGGAAATTTTGGGGACCAGGAA CTAATCAGACAAGGAACTGATTACAAACATTGGCCGCAAATTGCACAATTTGCCCCCAGCGC TTCAGCGTTCTTCGGAATGTCGCGCATTGGCATGGAAGTCACACCTTCGGGAACGTGGTTGA CCTACACAGGTGCCATCAAATTGGATGACAAAGATCCAAATTTC (SEQ ID NO: 3) |
| Influenza A virus (GenBank: MG027914.1, A/C aliform a/M A 07/2009(H1N1), HA gene) |
| CCGGGAGACAAAATAACATTCGAAGCAACTGGAAATCTAGTGGTACCGAGATATGCATTCG CAATGGAAAGAAATGCTGGATCTGGTATTATCATTTCAGATACACCAGTCCACGATTGCAAT ACAACTTGTCAGACACCCAAGGGTGCTATAAACACCAGCCTCCCATTTCAGAATATAC (SEQ ID NO: 4) |
| Influenza B virus (GenBank: CY018366.1, B/Florida/02/2006, M1 gene) |
| ATGTCGCTGTTTGGAGACACAATTGCCTACCTGCTTTCATTGACAGAAGATGGAGAAGGCAA AGCAGAACTAGCAGAAAAATTACACTGTTGGTTCGGTGGGAAAGAATTTGACCTAGACTCT GCTTTGGAATGGATAAAAAACAAAAGATGC (SEQ ID NO: 5) |

**[Table 4]**

| SEQ ID NOS: 6 to 11: Primers for target sequence amplification (primers targeted for RPA) | |
|---|---|
| Covid-19 (GenBank: MW811435.1, SARS-CoV-2/human/USA/USA-WA1/2020, N gene) | |
| Forward | 5'-AAC ACA AGC TTT CGG CAG-3'(SEQ ID NO: 6) |
| Reverse | 5'-GAA ATT TGG ATC TTT GTC ATC C-3' (SEQ ID NO: 7) |
| Influenza A virus (GenBank: MG027914.1, A/California/MA_07/2009(H1N1), HA gene) | |
| Forward | 5'-CCG GGA GAC AAA ATA ACA TTC-3' (SEQ ID NO: 8) |
| Reverse | 5'-GTA TAT TCT GAA ATG GGA GGC-3' (SEQ ID NO: 9) |
| Influenza B virus (GenBank: CY018366.1, B/Florida/02/2006, M1 gene) | |
| Forward | 5'-ATG TCG CTG TTT GGA GAC ACA ATT G-3' (SEQ ID NO: 10) |
| Reverse | 5'-GCA TCT TTT GTT TTT TAT CCA TTC-3' (SEQ ID NO: 11) |

**[Table 5]**

| SEQ ID NOS: 12 to 14: Information on guide RNAs |
|---|
| Covid-19 (GenBank: MW811435.1, SARS-CoV-2/human/USA/USA-WA1/2020, N gene) |
| 5'-UAA UUU CUA CUA AGU GUA GAU CCC CCA GCG CUU CAG CGU UC-3'-NH₂ (SEQ ID NO: 12) |
| Influenza A virus (GenBank: MG027914.1, A/California/MA_07/2009(H1N1), HA gene) |
| 5'-UAA UUU CUA CUA AGU GUA GAU AGA UAC ACC AGU CCA CGA UU-3'-NH₂ (SEQ ID NO: 13) |
| Influenza B virus (GenBank: CY018366.1, B/Florida/02/2006, M1 gene) |
| 5'-UAA UUU CUA CUA AGU GUA GAU AUU GAC AGA AGA UGG AGA AG-3'-NH₂ (SEQ ID NO: 14) |

**[Table 6]**

| SEQ ID NOS: 15 and 16: Reporter DNAs for fluorescence signal detection | | |
|---|---|---|
| 1 | Reporter DNA for QC (quencher attached) | 5'-TAMRA-TTA TTA TT-3'-BHQ2 (SEQ ID NO: 15) |
| 2 | Reporter DNA-for fluorophore attachment | 5'-Thiol-TTA TTA TT-3'-NH₂ (SEQ ID NO: 15) |
| 3 | Reporter DNA-TAMRA | 5'-NH₂-TTA TTA TTT T-3'-TAMRA (SEQ ID NO: 16) |

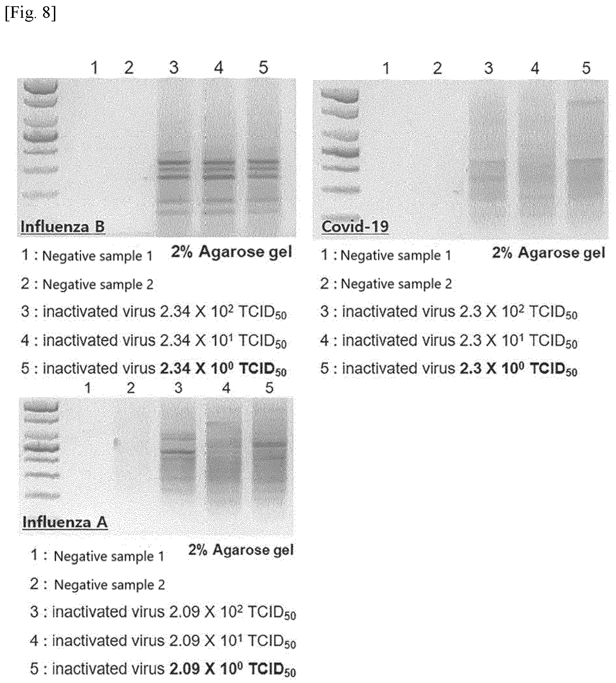

Fig. 8 shows the results of capture and amplification of nucleic acids from the inactivated viruses as targets. The inactivated viruses had the target sequences set forth in SEQ ID NOS: 2 and 3. The inactivated viruses were lyzed and nucleic acids were captured therefrom. Referring to Fig. 8, the target nucleic acids were normally moved between the wells and were amplified by RT-RPA enzyme complexes. In Fig. 8, lanes 1 and 2 show the results of RT-RPA after the same process was performed without the samples and lanes 3 to 5 show the results of RT-RPA based on the indicated tissue culture infective dose 50% (TCID₅₀) of coronavirus (Covid-19), influenza A virus, and influenza B virus. TCID₅₀ represents the viral load at which 50% of cells are infected during cell culture. The electrophoresis data reveal that viral targets with low TCID₅₀ values of 2.34, 2.3, and 2.09 can be lyzed and target nucleic acids can be amplified through RT-RPA, indicating that the magnetic particle probes are expected to sufficiently detect target nucleic acids with high sensitivity.

Based on the above results, it can be indirectly suggested that target signals from the amplified nucleic acids can be detected.

### Example 3. Construction of the magnetic particle probes for signal detection and confirmation of their functionality (Well 4 and Well 5)

In the magnetic particle probes for signal detection, the guide RNAs (gRNAs) play a role in recognizing the target sequences of the target factor and the gRNA/target sequence/Cas12a complexes play a role in cleaving the sequences. Here, the magnetic particle probes were constructed in which the single-stranded reporter DNAs responsible for signal detection were mixed. Therefore, it is necessary to investigate whether the gRNAs playing a role in recognizing the target sequences when they were mixed with the reporter DNAs and coupled to the magnetic particles.

An experiment was designed to confirm the results obtained when the gRNAs or the fluorescence factor-attached reporter DNAs were coupled to the magnetic particles. Information on the sequences of the gRNAs and the reporter DNAs can be found in SEQ ID NOS: 4 and 5 in the sequence listing.

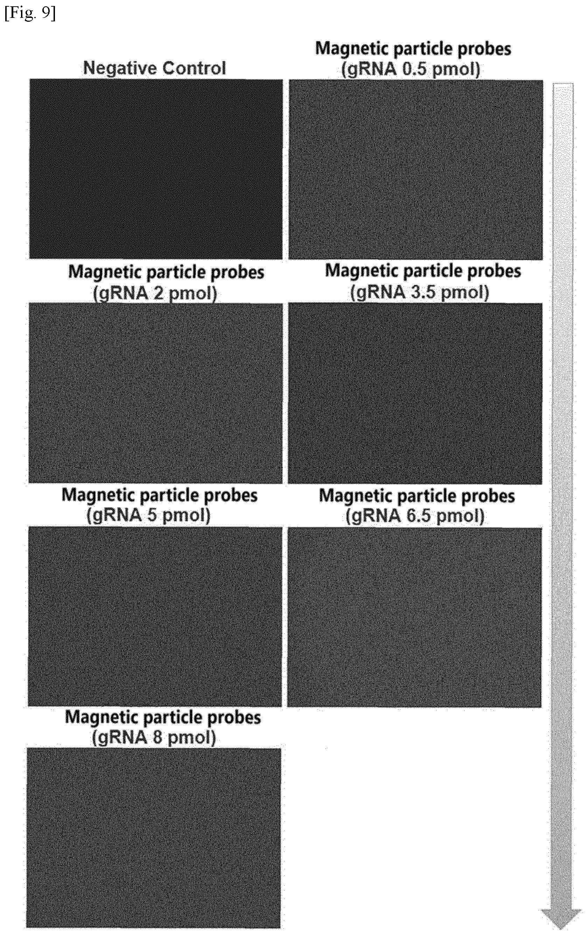

Fig. 9 confirms concentration-dependent changes in the activity of a Cas system by magnetic particles coupled with different concentrations of gRNAs in a state in which free reporter DNAs and a Cas protein were mixed, in the presence of target nucleic acids. A test was conducted to confirm the concept of the Cas system. To this end, a fluorescence factor was bound to one side of each of the free reporter DNAs and a quencher absorbing fluorescence signals was bound to the other side. Referring to Fig. 9, when different concentrations of the gRNA sequences were coupled to the surface of the magnetic particles, the quencher of the free reporter DNAs, where the functionality of the fluorescence factor was masked by the quencher, was cut out depending on the concentration of the gRNAs, resulting in the detection of fluorescence signals.

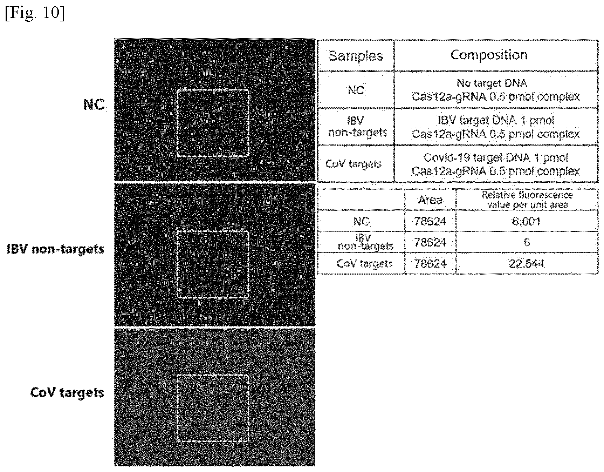

Fig. 10 shows the results of an experiment for confirming the activity of the Cas system for non-target nucleic acids.

Referring to Fig. 10, no fluorescence signals were observed from the Covid-19-targeted magnetic particle probes coupled with Covid-19 gRNAs because the Cas protein was not activated in the presence of non-target sequences of influenza B virus. These results indirectly demonstrate the target-specific functionality of gRNA/Cas12a.

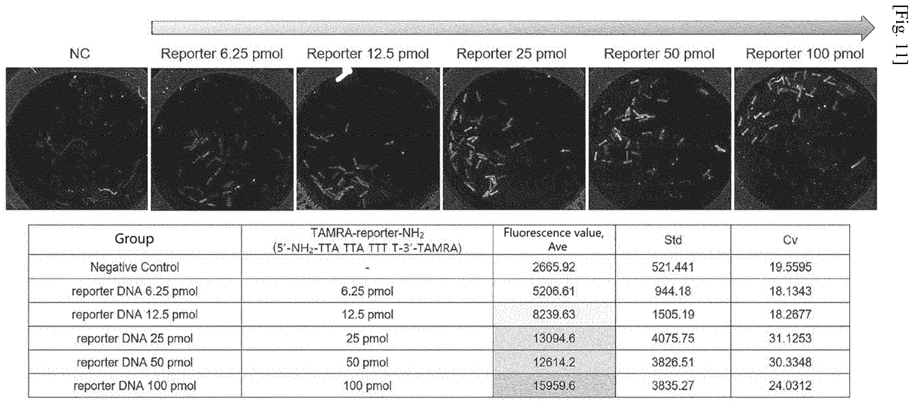

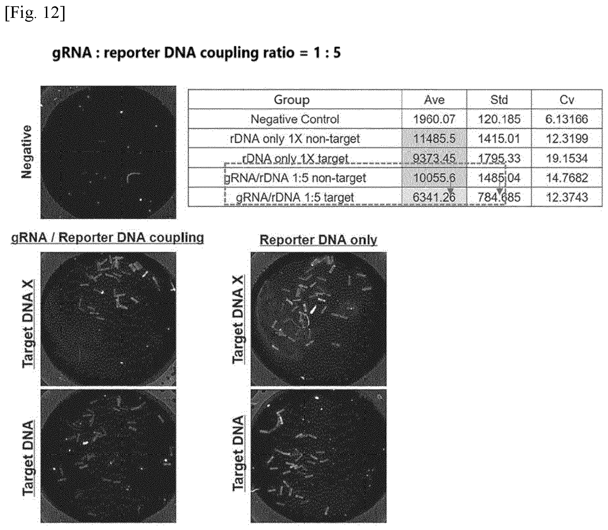

Fig. 11 shows the results of a test for coupling of the magnetic particles with optimal concentrations of the reporter DNAs. Referring to Fig. 11, when the fluorescence factor-attached reporter DNA sequences were individually coupled to the magnetic particles, a similar level of fluorescence signals was observed even at a low coupling concentration of 25 pmol compared to the levels of fluorescence signals observed at coupling concentrations of 50 and 100 pmol. As a result, the final coupling concentration conditions of the gRNAs and the reporter DNAs were determined. Subsequently, the gRNAs and the reporter DNAs were mixed at the coupling concentrations determined in Fig. 11 and were coupled to the magnetic particles. Thereafter, a decrease in fluorescence signal depending on the presence or absence of the target sequences was investigated. Fig. 12 reveals that fluorescence signals from the magnetic particle probes coupled with the gRNAs and the reporter DNAs decreased due to the activity of the Cas system activity in the presence of target nucleic acids. Referring to Fig. 12, the fluorescence signals from the magnetic particles coupled with the mixture of the gRNAs and the reporter DNAs were significantly reduced when the target sequences were present. In contrast, the change in fluorescence signal from the magnetic particles coupled with the reporter DNAs only was insignificant depending on the presence or absence of the target sequences. These results demonstrate that the magnetic particle probes including the gRNAs and the reporter DNAs can detect target sequences of the target factor.

### Example 4. Implementation of multiple diagnostics based on the magnetic particle probes

Based on the results of the previous examples confirming the ability of the magnetic particle probes to detect target factors, an investigation was made as to whether the magnetic particle probes can diagnose a single target factor and differentially diagnose a plurality of target factors.

Coronavirus (Covid-19)-targeted magnetic particle probes were constructed to confirm signal detection for the single target factor. The detection of signals from the magnetic particle probes for coronavirus (Covid-19), influenza A virus, and influenza B virus targets was confirmed.

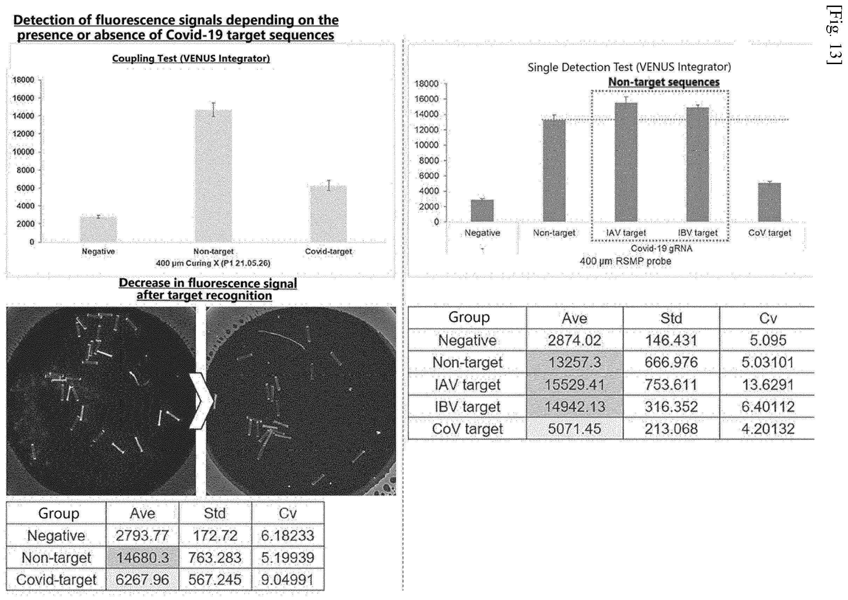

After all reactions proceeded in a single process in Well 1 to Well 8, the intensities of detected fluorescence signals were measured. Referring to the results on the left side of Fig. 13, the intensity of fluorescence signals (fluorescence value = 6267.96) from the magnetic particle probes coupled with Covid-19-targeted gRNAs in the Covid-target group where coronavirus (Covid-19) was present as a target was significantly reduced compared to that (fluorescence value = 14680.3) in the non-target group where target nucleic acids were not present, demonstrating a difference in fluorescence signal after the target-specific detection of Covid-19 target nucleic acids by the magnetic particle probes. The negative group relates to magnetic particle probes uncoupled with gRNAs and reporter DNAs. Referring to the results on the right side of Fig. 13, the intensity of fluorescence signals (fluorescence value = 5071.45) from the magnetic particle probes coupled with the Covid-19-targeted gRNAs in the CoV target group where coronavirus (Covid-19) was present as a target was significantly reduced compared to those in the non-target group where target sequences were not present (fluorescence value = 13257.3), the IAV target group where influenza A virus target sequences were present (fluorescence value = 15529.41), and the IBV target group where influenza B virus target sequences were present (fluorescence value = 14942.13). These results concluded that the differences in fluorescence signal from the Covid-19-targeted magnetic particle probes are because the Cas12a enzyme complexes are activated only in the presence of coronavirus (Covid-19) and confirmed that the Covid-19-targeted magnetic particle probes can be used for target-specific diagnosis.

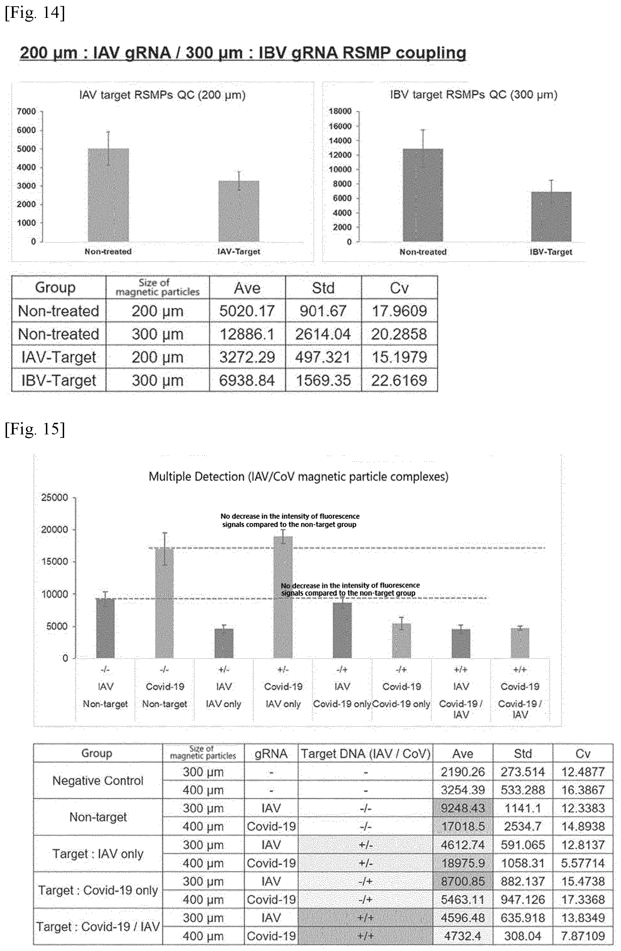

The applicability of the magnetic particle probes to target-specific diagnosis can be further confirmed in Fig. 14. Referring to the results on the left side of Fig. 14, the intensity of fluorescence signals (fluorescence value = 3272.29) from the 200 µm long magnetic particles coupled with influenza A-targeted gRNAs and reporter DNAs in the IAV-target group where influenza A virus target sequences were present was reduced compared to that (fluorescence value = 5020.17) in the non-treated group where influenza A virus target sequences were not present. Referring to the results on the right side of Fig. 14, the intensity of fluorescence signals (fluorescence value = 6938.84) from the 300 µm long magnetic particles coupled with influenza B-targeted gRNAs and reporter DNAs in the IBV-target group where influenza B virus target sequences were present was reduced compared to that (fluorescence value = 12886.1) in the non-treated group where influenza B virus target sequences were not present. These results concluded that the differences in fluorescence signal from the targeted magnetic particle probes are because the Cas12a enzyme complexes are activated only in the presence of suitable target sequences and reconfirmed that the magnetic particle probes can be used for target-specific diagnosis.

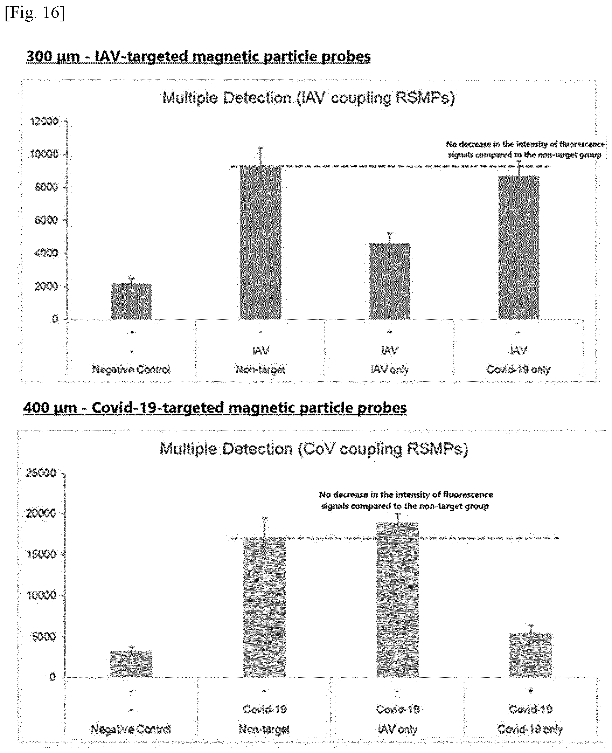

An experiment was conducted to confirm that the magnetic particle probes of the present invention can perform multiple diagnostics in the presence of multiple targets. For this purpose, Covid-19-targeted 400 µm magnetic particle probes (orange bars in Fig. 15) and 300 µm influenza A-targeted magnetic particle probes (blue bars in Fig. 15) were prepared and used for multiple detection of multiple targets. The results of graph analysis shown in Figs. 15 and 16 reveal that the magnetic particle probes of the present invention can play a role in recognizing the presence of either one or two targets.

## Claims

1. Microparticle probes for isolating and detecting nucleic acids to detect target nucleic acids, each of the microparticle probes comprising: a microparticle; capture probes introduced on the surface of the microparticle and comprising sequences complementary to those of the target nucleic acids; and reporter nucleic acids introduced on the surface of the microparticle and generating signals in response to an external stimulus.

2. The microparticle probes according to claim 1, wherein the microparticle contains magnetic particles.

3. The microparticle probes according to claim 1, wherein the microparticle has a core-shell structure in which a shell layer having a uniform thickness surrounds a core comprising a magnetic material.

4. The microparticle probes according to claim 1, wherein the microparticle has a size and specific gravity such that it is not suspended in water.

5. The microparticle probes according to claim 1, wherein the capture probes comprise nucleotide sequences for the application of gene editing technology.

6. The microparticle probes according to claim 1, wherein the capture probes comprise guide RNAs.

7. The microparticle probes according to claim 6, wherein each of the guide RNAs comprises a CRISPR RNA (crRNA) having a nucleotide sequence capable of hybridizing with a target site of a gene and a trans-activating crRNA (tracrRNA).

8. The microparticle probes according to claim 6, wherein the guide RNAs bind to the target nucleic acids and can cleave the target nucleic acids and the reporter nucleic acids when they encounter a restriction enzyme.

9. The microparticle probes according to claim 1, wherein the reporter nucleic acids are labeled with a luminescent material.

10. The microparticle probes according to claim 1, wherein the reporter nucleic acids are nucleotide sequences to be cleaved when the microparticle probes are utilized in a gene editing system.

11. A kit for detecting target nucleic acids, the kit comprising the microparticle probes for nucleic acid isolation and detection according claims 1, a restriction enzyme, and a reagent for nucleic acid amplification.

12. The kit according to claim 11, wherein the restriction enzyme is a Cas endonuclease.

13. A method for detecting target nucleic acids, the method comprising (a) extracting target nucleic acids from a sample, (b) amplifying the target nucleic acids, (c) providing microparticle probes for isolating and detecting the target nucleic acids, each of the microparticle probes comprising: a microparticle; guide RNAs introduced on the surface of the microparticle and comprising sequences complementary to those of the target nucleic acids; and reporter nucleic acids introduced on the surface of the microparticle and generating signals in response to an external stimulus, (d) allowing the guide RNAs of the microparticle probes for target nucleic acid isolation and detection to react with the target nucleic acids to form complexes of the microparticle probes for target nucleic acid isolation and detection and the target nucleic acids, (e) allowing a restriction enzyme to bind to the guide RNAs of the microparticle probes for target nucleic acid detection, (f) activating the restriction enzyme to cleave the nucleotide sequences of the target nucleic acids and the reporter nucleic acids, and (g) measuring on/off of signals emitted from the complexes in response to an external stimulus to detect the target nucleic acids.

14. The method according to claim 13, wherein the two or more types of microparticle probes for nucleic acid isolation and detection are used to detect the two or more types of target nucleic acids.

15. The method according to claim 13, wherein the two or more types of microparticle probes for nucleic acid isolation and detection are labeled with length, diameter, thickness, shape, color or identification codes so as to be distinguished from each other.

16. The method according to claim 13, wherein the microparticle probes for nucleic acid isolation and detection are in the form of microrods.

17. A multiple diagnostic method for isolating and detecting target nucleic acids in a sample using genetic scissors introduced to the microparticle probes for nucleic acid isolation and detection according claims 1, the method comprising: amplifying target nucleic acids in a sample and complementarily binding the target nucleic acids to the microparticle probes for nucleic acid isolation and detection; and allowing genetic scissors introduced to the microparticle probes for nucleic acid isolation and detection to cleave the surrounding reporter nucleic acids when the genetic scissors recognize the complementary binding, wherein a decrease in signal by the cleavage of the reporter nucleic acids is measured to detect the target nucleic acids.

18. The multiple diagnostic method according to claim 17, wherein the genetic scissors is a CRISPR-Cas system.

19. The multiple diagnostic method according to claim 17, wherein the signal is fluorescence or chemiluminescence.
